# EUROPEAN PATENT SPECIFICATION

(11) **EP 3 503 756 B1**
(45) Date of publication and mention of the grant of the patent: **11.03.2026**
(21) Application number: 18725996.5
(22) Date of filing: 01.05.2018
(51) Int. Cl.: A43B 3/00, A43B 7/14, A43B 7/24

(54) **MIDSOLE WITH INCORPORATED ORTHOTIC SUPPORT**
ZWISCHENSOHLE MIT INTEGRIERTER ORTHOPÄDISCHER UNTERSTÜTZUNG
SEMELLE INTERMÉDIAIRE AVEC SUPPORT ORTHOPÉDIQUE INCORPORÉ

(30) Priority: 20.07.2017 US 201715655624
(43) Date of publication of application: 03.07.2019
(73) Proprietor: Superfeet Worldwide LLC, Ferndale, WA 98248 (US)
(72) Inventor: HAYES, Eric Paris, Ferndale WA 98248 (US); ZHUANG, Mike, Ferndale WA 98248 (US); WAKELAND, Daniel, Ferndale WA 98248 (US)
(74) Representative: Hoffmann Eitle
(86) International application number: PCT/US2018/030478
(87) International publication number: WO 2019/018044

(56) References cited:
- EP-A1- 3 045 066
- WO-A1-96/38062
- US-A- 4 831 750
- US-B2- 9 259 050

## Description

### FIELD OF THE INVENTION

This disclosure generally relates to footwear, and more particularly to footwear with orthotic devices incorporated into sole assemblies.

### BACKGROUND OF THE INVENTION

Footwear is often designed without proper support for the human foot. This is especially true if the foot is affected by one of many common ailments. This lack of support is often addressed by orthotic inserts (also referred to as "orthotics"), that are devices placed in footwear to cooperate with the plantar surfaces of a wearer's feet. The inserts enhance one or both of comfort and support.

The ability to remove orthotic inserts is advantageous in certain situations because it allows wearers to conveniently switch inserts from one pair of footwear to another. However, removable inserts can be easily misplaced or lost. In addition, orthotic inserts can become dislodged or misaligned during use, thereby diminishing their effectiveness. Many orthotic inserts are designed as aftermarket products that must fit a wide variety of footwear. The design of the orthotic is limited by conforming to generic footwear. For example, many orthotic inserts must be designed with a limited height to allow them to fit into the foot compartment of footwear.

WO 96/38062 discloses an insert for a shoe sole including a heel lever which absorbs energy from the foot during heel strike and returns the energy to the foot during heel lift-off.

US 9 259 050 B2 relates to sole structures incorporating a rigid support structure that cradles the heel of the user that comprises a plurality of curvatures.

### SUMMARY OF THE INVENTION

The object of the invention is to provide a sole structure for an article of footwear according to claim 1, a method of manufacturing said structure according to claim 6 and an article of footwear incorporating said structure according to claim 11, with preferred embodiments in the dependent claims 2-5, 7-10 and 12-14, respectively.

Footwear with orthotic support incorporated into the sole assembly provide the support of orthotic inserts but in a fixed and durable package. An orthotic support incorporated within an article of footwear cannot shift while in use or be lost by a user. The incorporation of orthotics into the sole assembly creates many design possibilities that cannot be realized with removable orthotic inserts. The footwear with incorporated orthotic provide proper support when purchased by the consumer without having to add an insole or orthotic after purchase of the footwear.

At least one embodiment of the invention may be summarized as an orthotic support incorporated within a midsole. The midsole is formed of a soft and flexible material that surrounds and holds the orthotic support in position. The orthotic features a heel cup and curvature to support the arch of a foot.

The orthotic features portions that are shaped to interact with particular surfaces of a foot. The heel cup supports the heel of a user. The curvature of the cup compliments the curvature of a heel and contributes to creating a proper fit. The orthotic extends forward through the arch of the foot and has curvature that supports the arch. The curvature of the heel cup and arch support can be designed to fit a specific user, foot ailment, or other parameter. One embodiment of the invention includes an orthotic support with a length that extends from the back of the heel cup through the arch support. This length allows flexibility of the footwear forward of the orthotic. Other embodiments may include orthotics that are approximately the length of the midsole. Different materials or thicknesses can be used along the length, width, and thickness of the orthotic to allow desired flexibility and support in various regions.

The orthotic may increase the rigidity of the footwear. It is preferably composed of a material that is substantially more rigid than the flexible material surrounding it. One embodiment uses nylon, a material commonly used for orthotics, but many others may be used. The rigidity of the material provides support to the plantar surfaces of the foot. This support contributes to optimal functioning of a foot. The orthotic may increase one or both of the flexural and torsional strength of the midsole. The rigidity of the orthotic support may be designed to provide proper support for specific foot ailments, foot shapes, or other parameters.

The rigidity of footwear may also be increased using shanks. Shanks are commonly linear metal components that are positioned approximately parallel to the longitudinal axis of footwear. In one embodiment of the invention, a shank may be incorporated into the midsole with an orthotic insert to provide further rigidity. In other embodiments, the orthotic support may provide the rigidity of a traditional shank.

The rigidity of the footwear may also be designed for by altering other aspects of the footwear. In one embodiment, the thickness of the orthotic may be increased in high stress areas. Other embodiments may use stiffer materials or shape the orthotic to increase rigidity. Embodiments may also include flexible areas, such as flex grooves, that align with bending points in the foot. These flexible areas may be designed by reducing thickness or reducing cross sectional area. Some embodiments may choose material rigidity based on the application of the footwear. Other embodiments may use multiple materials with different rigidities to form a midsole.

The material that surrounds the orthotic is preferably substantially softer and more flexible than the orthotic. A common material used in this type of application is polyurethane but many other materials may be used. This soft and flexible material holds the orthotic in position. Some embodiments feature hollow space within the orthotic thatthe soft and flexible material may passes through. The material that passes through the hollow space further secures the orthotic in position. The shape, thickness, and rigidity of the soft and flexible material may be varied, similarly to the orthotic. Embodiments may include a midsole with increased thickness of the flexible material at locations that require high rigidity. Embodiments may also include decreased thickness of the material in areas that require flexibility, such as the toe box. Some embodiments may feature midsoles with different rigidity based on the intended use of the footwear. An embodiment of an athletic shoe may feature a more flexible midsole material than an embodiment of a work boot.

Midsoles come in a wide variety of shapes according to the intended use of the footwear. Embodiments will likewise vary in shape.

The midsole may be produced in several ways. In one embodiment, the orthotic support is held in place by structures extending from the bottom surface of the upper portion of the footwear. These structures hold the orthotic support in a fixed position between the upper portion and an outsole of the footwear. A material then flows into the space between the upper portion and the outsole, surrounding the orthotic support. This material cures forming a midsole with the orthotic support incorporated within it. Other embodiments may feature the orthotic support being held in place by structures extending from the outsole. In other embodiments, the upper portion of the footwear may be replaced by other surfaces. Further embodiments may replace the outsole with a different lower surface.

Some embodiments include hollow space in the orthotic support that allows the flowing material to pass through it. This material subsequently cures and contributes to securing the orthotic support in position.

The method of producing the midsole may also affect the rigidity of the sole assembly. In an embodiment, the material reaction rate may be varied with time producing different thicknesses along the length of the midsole of the footwear. Varying the thickness allows for the creation of specific areas of flexibility and rigidity. In further embodiments, a shank may also be incorporated into the midsole. The shank may be positioned below the orthotic. The flowing material would surround the shank and orthotic incorporating them into the midsole.

### BRIEF DESCRIPTION OF THE DRAWINGS

Preferred and alternative examples of the present invention are described in detail below with reference to the following drawings:
Figure 1 shows a side view of an embodiment of a midsole.
Figure 2 shows an exploded view of the orthotic insert, soft and flexible material, upper portion of the footwear, and outsole.
Figure 3 shows a top view of the orthotic, including hollow space, and the flexible material.
Figure 4 shows an embodiment of a method of production.
Figure 5 shows a midsole encapsulating both an orthotic and a shank.

### DETAILED DESCRIPTION

In the following description, certain specific details are set forth in order to provide a thorough understanding of various disclosed embodiments. However, one skilled in the relevant art will recognize that embodiments may be practiced without one or more of these specific details. In other instances, well known structures and manufacturing techniques associated with footwear and orthotic devices may not be shown or described in detail to avoid unnecessarily obscuring descriptions of the embodiments.

Unless the context requires otherwise, throughout the specification and claims that follow, the word "comprise" and variations thereof, such as, "comprises" and "comprising" are to be construed in an open, inclusive sense, that is as "including, but not limited to."

Unless otherwise stated, the following terms shall refer to the stated descriptions. The length of the footwear shall refer to an axis extending from the heel portion of the footwear through the toe portion of the footwear. The width of the footwear shall refer to an axis approximately perpendicular to the length axis and approximately parallel to the bottom surface of the footwear. The thickness of the footwear shall refer to an axis perpendicular to the length and width of the footwear.

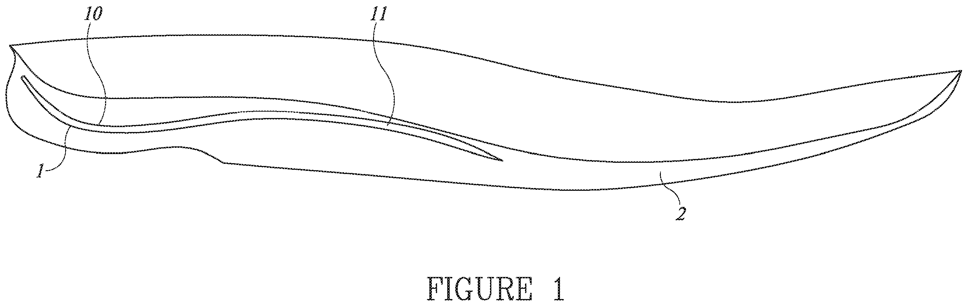

Figure 1 depicts a side view cross section of an embodiment of a footwear midsole. The figure depicts an orthotic insert 1 surrounded by soft and flexible material 2. The heel cup 10 is shown in the rear of the footwear. The arch support portion 11 is shown extending forward from the rear portion. One embodiment of the curvature of the arch support portion 11 is shown in Figure 1. However, the arch support portion of the orthotic could be formed using many different curvatures to support different types and/or shapes of feet or alleviate different foot ailments.

Figure 1 also depicts an embodiment of a possible length of an orthotic support. Figure 1 depicts the orthotic insert terminating after the arch support. This configuration allows for increased flexibility of the midsole member in the forward portions of the footwear. Other embodiments may include different lengths to provide support to other areas of the foot.

Figure 1 also depicts an embodiment of the invention with varying thickness of the midsole. The forward portion of the midsole is depicted as having less cross-sectional thickness than the rearfoot or middle portions of the midsole. A thinner forward portion of the midsole may provide greater flexibility. Higher flexibility is desirable in applications including athletic shoes. Other embodiments may vary thickness differently than depicted in Figure 1. The thickness variation may be based on intended application of the footwear or other design parameters.

The thickness of the orthotic insert may also be varied. The thickness variation of the orthotic insert may be varied in conjunction with or independently of the thickness variation of the flexible material. Figure 1 depicts varying the thickness of both the orthotic insert and flexible material. The orthotic insert 1 is illustrated to have a tapered thickness as it extends to the front of the footwear. The flexible material 2 has greater thickness in the heel portion of the footwear. Other embodiments of the invention may use thickness variation of the components to provide support in different areas of the footwear.

Varying the thickness of the midsole members allows for the characteristics of the midsole to be designed for specific areas of a foot. One embodiment of the invention may include a thicker section of the arch support portion of the orthotic insert. This thicker section may provide support to a specific area of the foot to correct for a foot ailment. Another embodiment may include thicker flexible material in the rearfoot portion of the midsole to provide great shock absorption. The thickness of both members may be varied in different portions of the footwear to tailor the characteristics of the footwear to an intended purpose, a specific foot, a specific foot ailment, or other design parameters.

The characteristics of the midsole may be varied in other ways. An embodiment of the invention may include an orthotic insert composed of multiple materials with different rigidities. Other embodiments may use an orthotic insert material to provide a rigidity based on the intended use of the footwear. Embodiments may also vary the shape of the orthotic insert. One embodiment may reduce the width of the orthotic insert to provide greater torsional flexibility. Other preferred embodiments vary the thickness of the flexible material 2 above the orthotic 1. More cushion above the orthotic 1 can be used for a comfort application, while slightly less material above the orthotic or a firmer flexible material 2 above the orthotic may be used for a more performance-oriented application, for example.

Some embodiments of the invention may design the rigidity, thickness, material selection, shape, and other parameters based on the foot of a specific user. A user may have their foot measured by one or more of several methods such as a scan, dynamic pressure assessment, or plaster mold. These measurements may then be analyzed and used to create a footwear with support designed for a specific foot. Other embodiments may vary the characteristics of the midsole based on a specific foot ailment, shape, or other paramter. An example may include a midsole with an orthotic insert shaped to prevent overpronation of a foot. Still further embodiments may vary the design parameters of the footwear based on intended use. One embodiment may include a footwear designed for use as a work boot including a high degree of thickness of the midsole to provide support and shock absorption. Still further embodiments may design footwear based upon the analysis of the gait of an individual.

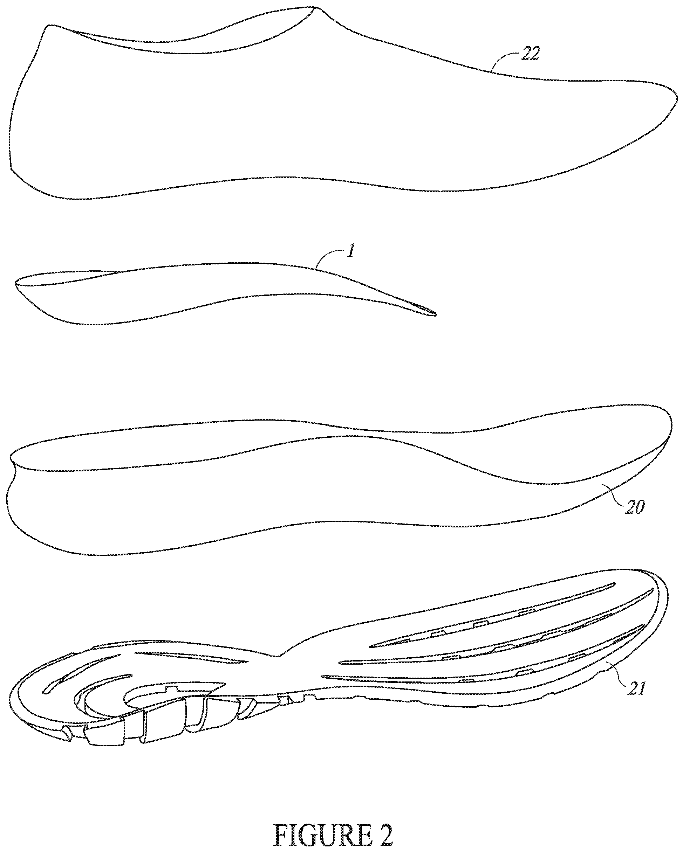

Figure 2 depicts an exploded view of the sole assembly of the footwear. This figure shows the orthotic insert 1 separately from a midsole member 20 composed of soft and flexible material 2. The figure also shows an outsole member 21 and upper portion 22.

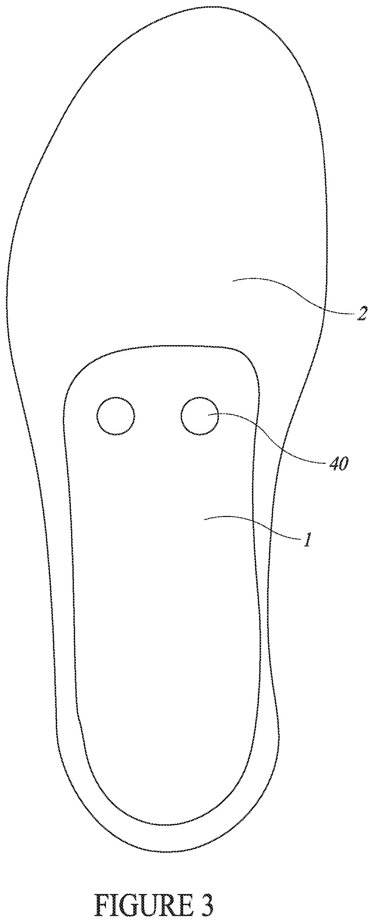

Figure 3 depicts a top view of an embodiment of a midsole of a footwear. This figure illustrates the shape of the orthotic 1 and soft and flexible material 2. Figure 3 depicts an embodiment of hollow spaces 40 passing through the thickness of the orthotic insert. Other embodiments may use a different number of hollow spaces, different shaped spaces, spaces differently oriented, or other variations. Some embodiments may include the soft and flexible material of the midsole passing through these hollow spaces 40. The material passing through the hollow space in the orthotic insert may help to hold the insert in place.

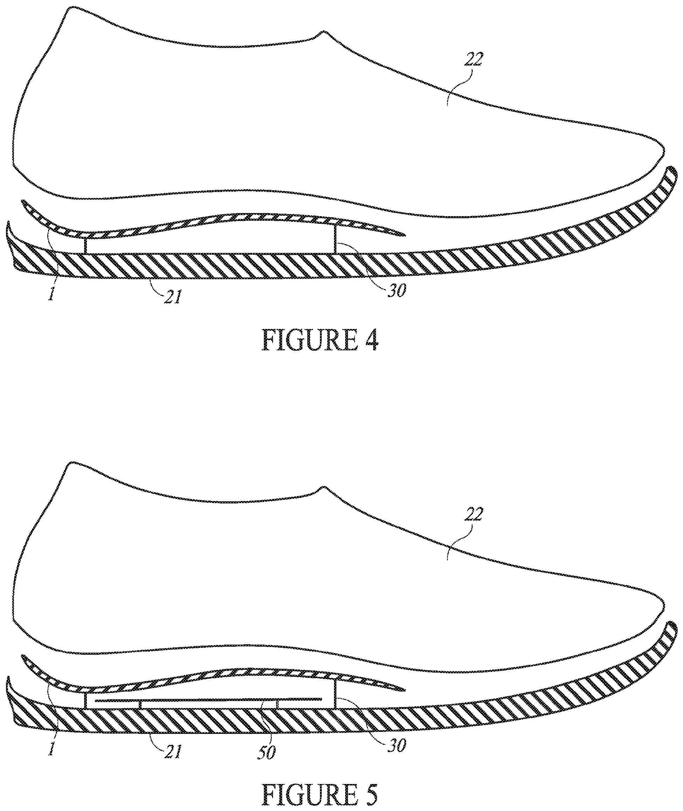

Figure 4 depicts a view of a configuration that may be used to produce the footwear. The figure depicts an upper portion of the footwear 22, an orthotic 1, and an outsole 21. The outsole is the portion of the footwear that contacts the ground. Figure 4 depicts an embodiment that holds the orthotic 1 in place a distance from the top surface of the outsole. This embodiment positions the orthotic using structures 30 extending from the top surface of the outsole. In this embodiment, the upper portion of the footwear is positioned such that the orthotic is between the outsole and upper portion. One embodiment of the invention includes injecting a flowing material into the space that surrounds the orthotic insert that is positioned between the outsole and upper portion. This material subsequently cures creating a midsole of the footwear with an incorporated, embedded orthotic. Other embodiments may include using structures extending from the bottom surface of the upper portion or extending from an outside structure to secure the orthotic in position.

Figure 4 depicts an orthotic insert with heel cup in the rearfoot portion of the footwear. Figure 4 also depicts an orthotic with a curvature that supports the arch of the foot. Some embodiments may include an orthotic with incorporated hollow space. Other embodiments may include the flowing material passing through these hollow spaces and subsequently curing within the hollow spaces.

Some embodiments may adjust the reaction rate of the material with time. This method could be used to vary the thickness along the length of the footwear. An embodiment may inject the material from the rear of the footwear and increase the reaction rate after a predetermined time. This embodiment could be employed to create a midsole with a rear portion that is thicker than the front portion. Other embodiments may vary the reaction rates in other ways to create thickness in a predetermined area of the footwear.

Figure 5 depicts an embodiment with an orthotic 1 and shank 50 incorporated into the midsole. The orthotic 1 and shank 50 are held in place between the upper portion of the footwear 22 and the outsole 21 by structures 30. A material is caused to flow into the space surrounding the orthotic 1 and shank 50. The material subsequently cures forming the midsole.

Shanks are commonly used in footwear to increase rigidity. An incorporated shank would be held in place by the soft and flexible material. One embodiment may include a rigid support member that is a long flat metal member approximately aligned with the lengthwise axis of the footwear. Other embodiments may include rigid support members comprised of different materials, of different shapes, or in different orientations. In some embodiments, the shank includes holes through that the flexible midsole material may flow during production.

## Claims

1. A sole assembly of an article of footwear having a heel portion, the sole assembly comprising;
a first midsole member (2) being substantially soft and flexible; and
a second midsole member (1) being substantially rigid and resilient, the second midsole member (1) being incorporated within the first midsole member (2), the second midsole member (1) having a rearfoot portion within the heel portion of the sole assembly, the rearfoot portion including a heel cup (10), the curvature of the cup compliments the curvature of a heel, the second midsole member (1) further having a forward portion that extends forwardly from the rearfoot portion and having curvature, and
the second midsole member (1) being surrounded by the first midsole member (2).

2. The sole assembly of claim 1 wherein:
the forward portion of the second midsole member extending forwardly through the approximate length of an arch support (11) and/or
the curved shape of the forward portion being concave relative to the bottom of the article of footwear.

3. The sole assembly of claim 1 wherein:
at least one of thickness and rigidity of the first and second members (2, 1) varies along the length of the footwear and/or
a rigid support member (50) being additionally incorporated within the first midsole member (2) below the second midsole member and/or
the second midsole member (1) being shaped to provide longitudinal and torsional rigidity.

4. The sole assembly of claim 1 wherein:
the curvature of the forward portion and angle of orientation of the forward portion relative to the horizontal transverse plane of the footwear being based on at least one of measurements of a foot or analysis of a gait.

5. The sole assembly of claim 1 wherein:
the second midsole member (1) having hollow space within it and the material of the first midsole member (2) passing through it.

6. A method of producing a midsole of an article of footwear having a heel portion wherein:
an orthotic (1) being held in a fixed position between an upper portion (22) of the article and an outsole (21), the orthotic (1) having a rearfoot portion within the heel portion of the sole assembly, the rearfoot portion including a heel cup (10), the curvature of the cup compliments the curvature of a heel, and the orthotic having a forward portion extending forwardly from the rearfoot portion and having curvature;
a material flows into a space between the upper portion (22) and outsole (21), surrounding the orthotic (1); and
the material curing to form a midsole member (2).

7. The method of claim 6 wherein:
the orthotic (1) having hollow space within it that allows for the material to flow through it.

8. The method of claim 6 wherein:
the orthotic (1) being held in place by support structures (30) extending from the bottom of the upper portion or by support structures extending from the upper surface of the outsole.

9. The method of claim 6 wherein:
a rigid support member (50) being additionally held in place below the orthotic (1) and/or
the orthotic (1) being shaped to provide longitudinal and torsional rigidity.

10. The method of claim 6 wherein:
the material reaction rate being adjusted to form varying thickness along the transverse plane of the article.

11. An article of footwear having at least an upper portion (22), an outsole (21), and a midsole (2), the midsole comprising:
an orthotic (1) having a heel end including a heel cup (10), the curvature of the cup compliments the curvature of a heel, the heel end having concave curvature relative to the top of the footwear;
a midsole material being (2) softer than the orthotic (1) and being positioned above and below the orthotic (1) such that the orthotic is suspended by the midsole (2) material between the upper (22) and the outsole (21); and
wherein the midsole material (2) surrounds the orthotic (1) such that it does not directly contact the outsole (21) or the upper (22).

12. The article of claim 11 wherein the orthotic (1) has hollow space within it.

13. The article of claim 11 wherein the orthotic (1) has a forward portion with convex curvature relative to the top of the footwear.

14. The article of claim 11 wherein a rigid support member (50) is surrounded by the midsole (2) material in addition to the orthotic.

## Patentansprüche

1. Sohlenbaugruppe eines Schuhwerks, das einen Fersenbereich aufweist, wobei die Sohlenbaugruppe umfasst:
ein erstes Zwischensohlenelement (2), das im Wesentlichen weich und flexibel ist; und
ein zweites Zwischensohlenelement (1), das im Wesentlichen starr und elastisch ist, wobei das zweite Zwischensohlenelement (1) innerhalb des ersten Zwischensohlenelements (2) eingebracht ist, wobei das zweite Zwischensohlenelement (1) einen Rückfußbereich innerhalb des Fersenbereichs der Sohlenbaugruppe aufweist, wobei der Rückfußbereich eine Fersenschale (10) einschließt, wobei die Krümmung der Schale der Krümmung einer Ferse komplementär ist, wobei das zweite Zwischensohlenelement (1) weiter einen vorderen Bereich aufweist, der sich vom Rückfußbereich nach vorne erstreckt und eine Krümmung aufweist, und
wobei das zweite Zwischensohlenelement (1) von dem ersten Zwischensohlenelement (2) umgeben ist.

2. Sohlenbaugruppe nach Anspruch 1, wobei
der vordere Bereich des zweiten Zwischensohlenelements sich in etwa über die Länge einer Fußgewölbestütze (11) nach vorne erstreckt und/oder
die gekrümmte Form des vorderen Bereichs relativ zur Unterseite des Schuhwerks konkav ist.

3. Sohlenbaugruppe nach Anspruch 1, wobei
mindestens eine der Eigenschaften Dicke oder Steifigkeit des ersten oder zweiten Zwischensohlenelements (2, 1) entlang der Länge des Schuhwerks variiert und/oder
ein starres Trägerelement (50) zusätzlich innerhalb des ersten Zwischensohlenelements (2) unterhalb des zweiten Zwischensohlenelements eingebracht ist und/oder
das zweite Zwischensohlenelement (1) so geformt ist, dass es Längs- und Torsionssteifigkeit bereitstellt.

4. Sohlenbaugruppe nach Anspruch 1, wobei
die Krümmung des vorderen Bereichs und der Orientierungswinkel des vorderen Bereichs relativ zur horizontalen Transversalebene des Schuhwerks auf mindestens einer der Messungen eines Fußes oder einer Analyse eines Gangs basieren.

5. Sohlenbaugruppe nach Anspruch 1, wobei
das zweite Zwischensohlenelement (1) einen Hohlraum innerhalb aufweist und das Material des ersten Zwischensohlenelements (2) diesen durchdringt.

6. Herstellungsverfahren einer Zwischensohle eines Schuhwerks, das einen Fersenbereich aufweist, wobei
ein orthopädischer Einsatz (1) in einer festen Position zwischen einem Schaftbereich (22) des Schuhwerks und einer Außensohle (21) gehalten wird, wobei der orthopädische Einsatz (1) einen Rückfußbereich innerhalb des Fersenbereichs der Sohlenbaugruppe aufweist, wobei der Rückfußbereich eine Fersenschale (10) einschließt, wobei die Krümmung der Schale der Krümmung einer Ferse komplementär ist, und wobei der orthopädische Einsatz einen sich vom Rückfußbereich nach vorne erstreckenden vorderen Bereich mit Krümmung aufweist;
ein Material in einen Zwischenraum zwischen dem Schaftbereich (22) und der Außensohle (21) einfließt und den orthopädischen Einsatz (1) umgibt, und
das Material aushärtet, um ein Zwischensohlenelement (2) auszubilden.

7. Verfahren nach Anspruch 6, wobei
der orthopädische Einsatz (1) einen Hohlraum innerhalb aufweist, der das Durchströmen des Materials ermöglicht.

8. Verfahren nach Anspruch 6, wobei
der orthopädische Einsatz (1) durch Trägerstrukturen (30), die sich von der Unterseite des Schaftbereichs erstrecken, oder durch Trägerstrukturen, die sich von der Oberseite der Außensohle erstrecken, in Position gehalten wird.

9. Verfahren nach Anspruch 6, wobei
ein starres Trägerelement (50) zusätzlich unterhalb des orthopädischen Einsatzes (1) in Position gehalten wird und/oder
der orthopädische Einsatz (1) so geformt ist, dass er Längs- und Torsionssteifigkeit bereitstellt.

10. Verfahren nach Anspruch 6, wobei
die Reaktionsgeschwindigkeit des Materials so eingestellt wird, dass sich entlang der Transversalebene des Schuhwerks unterschiedliche Dicken ausbilden.

11. Schuhwerk, das zumindest einen Schaftbereich (22), eine Außensohle (21) und eine Zwischensohle (2) aufweist, wobei die Zwischensohle umfasst
einen orthopädischen Einsatz (1) mit einem Fersenende, das eine Fersenschale (10) einschließt, wobei die Krümmung der Schale der Krümmung einer Ferse komplementär ist, wobei das Fersenende relativ zur Oberseite des Schuhwerks konkav gekrümmt ist,
ein Zwischensohlenmaterial (2), das weicher ist als der orthopädische Einsatz (1), und das oberhalb und unterhalb des orthopädischen Einsatzes (1) angeordnet ist, sodass der orthopädische Einsatz durch das Material der Zwischensohle (2) zwischen dem Schaft (22) und der Außensohle (21) aufgehängt ist, und
wobei das Zwischensohlenmaterial (2) den orthopädischen Einsatz (1) umgibt, sodass der orthopädische Einsatz nicht unmittelbar die Außensohle (21) oder den Schaft (22) berührt.

12. Schuhwerk nach Anspruch 11, wobei der orthopädische Einsatz (1) einen Hohlraum innerhalb aufweist.

13. Schuhwerk nach Anspruch 11, wobei der orthopädische Einsatz (1) einen vorderen Bereich mit konvexer Krümmung relativ zur Oberseite des Schuhwerks aufweist.

14. Schuhwerk nach Anspruch 11, wobei zusätzlich zu dem orthopädischen Einsatz ein starres Trägerelement (50) von dem Material der Zwischensohle (2) umgeben ist.

## Revendications

1. Ensemble de semelle d'un article chaussant présentant une portion talonnière, l'ensemble de semelle comprenant ;
un premier élément de semelle intermédiaire (2) étant sensiblement souple et flexible ; et
un deuxième élément de semelle intermédiaire (1) étant sensiblement rigide et résilient, le deuxième élément de semelle intermédiaire (1) étant incorporé au sein du premier élément de semelle intermédiaire (2), le deuxième élément de semelle intermédiaire (1) présentant une portion d'arrière-pied au sein de la portion talonnière de l'ensemble de semelle, la portion d'arrière-pied incluant une coque talonnière (10), la courbure de la coque complète la courbure d'un talon, le deuxième élément de semelle intermédiaire (1) présentant en outre une portion avant qui s'étend vers l'avant à partir de la portion d'arrière-pied et présentant une courbure, et
le deuxième élément de semelle intermédiaire (1) étant entouré par le premier élément de semelle intermédiaire (2).

2. Ensemble de semelle selon la revendication 1 dans lequel :
la portion avant du deuxième élément de semelle intermédiaire s'étend vers l'avant sur une longueur approximativement égale à celle d'un support de voûte plantaire (11) et/ou
la forme courbe de la portion avant étant concave par rapport à la face inférieure de l'article chaussant.

3. Ensemble de semelle selon la revendication 1 dans lequel :
au moins une grandeur parmi l'épaisseur et la rigidité des premier et deuxième éléments (2, 1) varie le long de la longueur de l'article chaussant et/ou
un élément de support rigide (50) étant en outre incorporé au sein du premier élément de semelle intermédiaire (2) sous le deuxième élément de semelle intermédiaire et/ou
le deuxième élément de semelle intermédiaire (1) étant façonné pour fournir une rigidité longitudinale et torsionnelle.

4. Ensemble de semelle selon la revendication 1 dans lequel :
la courbure de la portion avant et l'angle d'orientation de la portion avant par rapport au plan transversal horizontal de l'article chaussant étant basés sur au moins un élément parmi des mesures d'un pied ou une analyse d'une démarche.

5. Ensemble de semelle selon la revendication 1 dans lequel :
le deuxième élément de semelle intermédiaire (1) présentant en son sein un espace creux et le matériau du premier élément de semelle intermédiaire (2) le traversant.

6. Procédé de fabrication d'une semelle intermédiaire d'un article chaussant présentant une portion talonnière dans lequel :
un support orthopédique (1) étant maintenu en position fixe entre une portion supérieure (22) de l'article et une semelle d'usure (21), le support orthopédique (1) présentant une portion d'arrière-pied au sein de la portion talonnière de l'ensemble de semelle, la portion d'arrière-pied incluant une coque talonnière (10), la courbure de la coque complète la courbure d'un talon, et le support orthopédique présentant une portion avant qui s'étend vers l'avant à partir de la portion d'arrière-pied et présentant une courbure ;
un matériau s'écoule dans un espace entre la portion supérieure (22) et la semelle d'usure (21), entourant le support orthopédique (1) ; et
le matériau durcissant pour former un élément de semelle intermédiaire (2).

7. Procédé selon la revendication 6 dans lequel :
le support orthopédique (1) présentant en son sein un espace creux qui permet au matériau de s'écouler à travers lui.

8. Procédé selon la revendication 6 dans lequel :
le support orthopédique (1) étant maintenu en place par des structures de support (30) s'étendant à partir du dessous de la portion supérieure ou par des structures de support s'étendant à partir de la surface supérieure de la semelle d'usure.

9. Procédé selon la revendication 6 dans lequel :
un élément de support rigide (50) étant en outre maintenu en place sous le support orthopédique (1) et/ou
le support orthopédique (1) étant façonné pour fournir une rigidité longitudinale et torsionnelle.

10. Procédé selon la revendication 6 dans lequel :
la vitesse de réaction du matériau étant ajustée pour former une épaisseur variable le long du plan transversal de l'article.

11. Article chaussant présentant au moins une portion supérieure (22), une semelle d'usure (21), et une semelle intermédiaire (2), la semelle intermédiaire comprenant :
un support orthopédique (1) présentant une extrémité talonnière inclant une coque talonnière (10), la courbure de la coque complète la courbure d'un talon, l'extrémité talonnière présentant une courbure concave par rapport au dessus de l'article chaussant :
un matériau de semelle intermédiaire (2) plus souple que le support orthopédique (1) et étant positionné au-dessus et en-dessous du support orthopédique (1) de sorte que le support orthopédique est suspendu par le matériau de la semelle intermédiaire (2) entre le supérieur (22) et la semelle d'usure (21) ; et
dans lequel le matériau de semelle intermédiaire (2) entoure le support orthopédique (1) de sorte qu'il n'entre pas en contact direct avec la semelle d'usure (21) ou avec le supérieur (22).

12. Article chaussant selon la revendication 11 dans lequel le support orthopédique (1) présente en son sein un espace creux.

13. Article chaussant selon la revendication 11 dans lequel le support orthopédique (1) présente une portion avant avec une courbure convexe par rapport au dessus de l'article chaussant.

14. Article chaussant selon la revendication 11 dans lequel un élément de support rigide (50) est entouré par le matériau de la semelle intermédiaire (2) en plus du support orthopédique.
